# EUROPEAN PATENT SPECIFICATION

(11) **EP 3 466 477 B1**
(45) Date of publication and mention of the grant of the patent: **07.06.2023**
(21) Application number: 17802634.0
(22) Date of filing: 17.05.2017
(51) Int. Cl.: A61M 25/10, A61B 17/3207, A61B 17/22

(54) **BALLOON CATHETER AND METHOD FOR MANUFACTURING BALLOON BODY**
BALLONKATHETER UND VERFAHREN ZUR HERSTELLUNG DES BALLONKÖRPERS
CATHÉTER À BALLONNET ET PROCÉDÉ DE FABRICATION DE CORPS DE BALLONNET

(30) Priority: 26.05.2016 JP 2016104803
(43) Date of publication of application: 10.04.2019
(73) Proprietor: Goodman Co., Ltd., Aichi 460-0008 (JP)
(72) Inventor: FUJISAWA, Soichiro, Seto-shi Aichi 489-0976 (JP); IWANO, Kenshi, Seto-shi Aichi 489-0976 (JP); MIYAKE, Takamasa, Seto-shi Aichi 489-0976 (JP); OTA, Mitsuhiro, Seto-shi Aichi 489-0976 (JP); OGAWA, Tomokazu, Seto-shi Aichi 489-0976 (JP); OGAWA, Keisuke, Seto-shi Aichi 489-0976 (JP)
(74) Representative: Epping - Hermann - Fischer
(86) International application number: PCT/JP2017/018452
(87) International publication number: WO 2017/204042

(56) References cited:
- WO-A2-2007/065137
- JP-A- 2005 511 187
- JP-A- 2005 517 474
- JP-A- 2005 518 842
- JP-A- 2006 512 952
- JP-A- 2010 540 185
- JP-A- 2013 521 937
- US-A1- 2003 153 870
- US-A1- 2007 213 760
- US-A1- 2012 209 375
- US-A1- 2016 058 982
- US-B2- 7 524 321

## Description

### Technical Field

The present invention relates to a balloon catheter and a manufacturing method for a balloon body.

### Background Art

A balloon catheter is known that is used in treatments that dilate a blood vessel in a location where the vessel is constricted. A balloon catheter has been proposed that has a linear member on the surface of a balloon. The linear member acts on the blood vessel when the balloon is in an inflated state. For example, in a case where the inflating of the balloon causes the linear member to dig into an injured portion of the blood vessel, the balloon becomes resistant to sliding in relation to the injured portion. Therefore, by inflating the balloon, the balloon catheter can properly dilate the injured portion from the inner side of the blood vessel.

Patent Literature 1 discloses a balloon catheter that is provided with a balloon that has a plurality of wings as linear members. The plurality of the wings are relatively harder than the balloon. The plurality of the wings extend radially outward when the balloon is inflated. Therefore, the inflating of the balloon causes the plurality of the wings to exert strong pressure against the tissue of the blood vessel. Two methods for forming the plurality of the wings on the balloon have been suggested. The first is a method that forms a portion of the balloon into the plurality of the wings. The second is a method that uses a different material from the balloon and affixes the material to the balloon by welding, adhesion, fusing, or the like.

Patent Literature 2 discloses an expansion device for use in surgical procedures comprising: a balloon having a longitudinal axis and an expanded configuration, wherein a first cross-section of the balloon is substantially non-circular, wherein the first cross- section is substantially perpendicular to the longitudinal axis.

Patent Literature 3 relates generally to balloons and balloon catheters for internal body applications, and more particularly, to balloons having a plurality of lumens through which to access either the distal end of the catheter shaft or internal body-locations, and to methods of making such multi-lumen balloons.

Patent Literature 4 is directed to apparatuses and methods that can be used in the treatment of heart valve disease, including balloon valvuloplasty and the delivery of transcatheter heart valves.

Patent Literature 5 is related to medical balloons, in particular non-compliant medical balloons used with a balloon catheter in medical procedures such as angioplasty.

Patent Literature 6 relates to a balloon catheter for creating a longitudinal channel in a lesion and a method for constructing the balloon catheter.

Patent Literature 7 relates to a medical balloon and in some embodiments to a strengthened medical balloon having shaped and/or textured properties.

### Citation List

### Patent Literature

Patent Literature 1: Japanese Laid-Open Patent Publication No. 2005-511187
Patent Literature 2: WO 2007/065137 A2
Patent Literature 3: US 7 524 321 B2
Patent Literature 4: US 2012/209375 A1
Patent Literature 5: US 2007/213760 A1
Patent Literature 6: US 2003/153870 A1
Patent Literature 7: US 2016/058982 A1

### Summary of Invention

In a case where a linear member made from a different material from the balloon is joined to the balloon, the linear member tends to detach from the balloon as the balloon is inflated. In a case where the linear member has detached from the balloon, there is a possibility that the position of the linear member will shift away from the desired position. In a case where the linear member is not joined to the balloon, there is a possibility that the position of the linear member will shift away from the desired position as the balloon is inflated. In this manner, the positions of at least some of the linear members that are disposed on the outer circumferential surface of the balloon can shift away from their desired positions as the balloon is inflated.

An object of the present invention is to provide a balloon catheter and a manufacturing method for a balloon body that make it difficult for a linear member to shift away from its desired position on the balloon as the balloon is inflated.

The invention is defined by the claims. In the following, parts of the description and drawings referring to embodiments which are not covered by the claims are not presented as embodiments of the invention, but as examples useful for understanding the invention.

A balloon catheter according to a first aspect is characterized by including: a catheter shaft extending in an extension direction from a proximal end toward a distal end; a balloon provided on the catheter shaft and inflatable in a radially outward direction centered on the catheter shaft; a linear member disposed on at least a portion of an outer circumferential surface of the balloon and extending in the extension direction; and a pressing member pressing the linear member against the balloon.

According to the first aspect, the balloon catheter includes the pressing member, which presses the linear member against the balloon. Therefore, when the balloon is inflated, the balloon catheter is better able to inhibit the linear member from shifting position in relation to the balloon than would be the case if the linear member were attached to the balloon by welding, adhesion, fusing, or the like.

In the first aspect, the pressing member may include a membrane portion covering at least a portion of the outer circumferential surface of the balloon, and at least a portion of the linear member may be disposed between the membrane portion and the balloon. In this case, the balloon catheter is able to inhibit the linear member from shifting position in relation to the balloon by having the membrane portion press the linear member against the balloon. Making the pressing member that presses the linear member against the balloon in the form of a membrane makes it possible for the balloon catheter to use the pressing member to inhibit the diameter of the entire balloon from expanding. Accordingly, when the balloon moves through the interior of a blood vessel, the balloon catheter is able to inhibit the pressing member from snagging on the inner wall of the blood vessel. Therefore, the balloon catheter is able to inhibit any diminishment of the balloon's ability to pass through the blood vessel.

In the first aspect, the membrane portion may cover the entirety of the outer circumferential surface of the balloon. In this case, the balloon catheter, by covering the entire balloon with the membrane portion, can more appropriately inhibit the linear member from shifting position in a case where the balloon is inflated.

In the first aspect, the linear member may be disposed on at least an inflation region of the outer circumferential surface of the balloon, and the inflation region may be a region extending in the extension direction over a portion of the balloon, with a substantially constant diameter throughout the extension direction. In this case, the balloon catheter is able to cause the linear member to act appropriately on the blood vessel in the inflation region, which has a substantially constant diameter.

In the first aspect, the linear member may be provided with an inflation portion disposed on the inflation region of the balloon, and at least one of a distal end portion positioned toward the distal end from the inflation region and a proximal end portion positioned toward the proximal end from the inflation region. In this case, the balloon catheter is able to make the surface area of contact between the linear member and the outer circumferential surface of the balloon greater than it would be in a case where the linear member includes only the inflation portion. Therefore, in a case where the linear member is inhibited from shifting position by the portion where the linear member and the balloon are in contact, the balloon catheter is able to appropriately inhibit the linear member from shifting position due to the enlarging of the contact surface area.

In the first aspect, at least a portion of an outer side portion of an inner side portion and an outer side portion of the linear member may be harder than the balloon, the outer side portion being disposed on the opposite side of an inner side portion, the inner side portion facing the balloon. In this case, the balloon catheter is able to cause the linear member to act appropriately on the blood vessel when the balloon is inflated.

In the first aspect, the linear member may include an extendable soft portion having at least the inner side portion, and a hard portion having at least the outer side portion and having a hardness greater than that of the soft portion. In this case, the hardness of the hard portion of the linear member is harder than the hardness of the soft portion, so the linear member is able to cause the hard portion to act appropriately on the blood vessel when the balloon is inflated. The soft portion of the linear member can be elongated. Therefore, by elongating the soft portion in accordance with the inflating of the balloon, the balloon catheter is able to cause the linear member to be elongated in conjunction with the inflating of the balloon. Accordingly, when the balloon is inflated, the balloon catheter is able to inhibit the linear member from shifting position in relation to the balloon and from interfering with the inflating of the balloon.

In the first aspect, at least a portion of the linear member may be joined to the balloon. In this case, in addition to being pressed against the balloon by the pressing member, the linear member is directly joined to the balloon. Therefore, the balloon catheter is more appropriately able to inhibit the linear member from shifting position in relation to the balloon.

In the first aspect, the pressing member may include a joining portion disposed between the balloon and the linear member, and the linear member may be joined to the balloon by the joining portion. In this case, at the same time that it presses the linear member against the balloon, the pressing member can use the joining portion to join the linear member directly to the balloon.

In the first aspect, at least one of the distal end and the proximal end of the linear member may be joined to the catheter shaft. In this case, the balloon catheter is able to inhibit the linear member from shifting position in relation to the catheter shaft, at the same time that it can use the pressing member to inhibit the linear member from shifting position in relation to the balloon.

A manufacturing method according to a second aspect for manufacturing a balloon body, the balloon body including the balloon, the linear member, and the pressing member according to the first aspect. The method is characterized by processes including: disposing the linear member on the outer circumferential surface of the balloon; coating the balloon with a molten substance, with the balloon in a state in which the linear member is disposed on the outer circumferential surface, and the molten substance being the material of the pressing member in a molten state; and forming the pressing member by drying the molten substance that adheres to the balloon and the linear member after the coating process. In this case, the linear member is pressed firmly against the outer circumferential surface of the balloon by the pressing member. Accordingly, the balloon body can easily be manufactured such that the linear member does not readily shift position in relation to the balloon.

A manufacturing method according to a third aspect for manufacturing a balloon body, the balloon body including the balloon, the linear member, and the pressing member according to the first aspect. The method is characterized by processes including: disposing the pressing member along an inner wall of a die; disposing the linear member on the opposite side of the pressing member from the die; injecting a parison, which will become the basis for the balloon, on the opposite side of the pressing member from the linear member; and blowing air into the interior of the parison. The parison is pressed against the die by the air blown into the interior of the parison, thus forming the balloon and causing the pressing member and the linear member to adhere tightly to the balloon. In this case, the linear member is pressed firmly against the outer circumferential surface of the balloon by the pressing member. Accordingly, the balloon body can be manufactured such that the linear member does not readily shift position in relation to the balloon. The balloon body can be manufactured using the pressing member in its existing solid state. Accordingly, it is possible to inhibit any changes in the physical properties of the pressing member that might be due to changes in the state of the pressing member at the time of manufacturing.

A manufacturing method according to a forth aspect for manufacturing a balloon body, the balloon body including the balloon, the linear member, and the pressing member according to the first aspect. The method is characterized by processes including: disposing the linear member on the outer circumferential surface of the balloon; and affixing the membranous pressing member to the outer circumferential surface such that the pressing member covers at least a portion of the linear member from the outer side of the linear member. In this case, the balloon body can easily be manufactured such that the linear member is pressed against the outer circumferential surface of the balloon by the pressing member.

A manufacturing method according to a fifth aspect for manufacturing a balloon body, the balloon body including the balloon, the linear member, and the pressing member according to the first aspect. The method is characterized by processes including: disposing the linear member on the outer circumferential surface of the balloon; covering the linear member from the outer side of the linear member with the pressing member, which has a heat shrinking property; and causing the pressing member to shrink by heating the pressing member in the state in which the linear member is covered by the pressing member. In this case, it is possible to cause the pressing member to adhere tightly to the balloon and the linear member. Because this is a dry manufacturing method, the balloon body can be manufactured easily without requiring any large-scale equipment.

### Brief Description of Drawings

[Fig. 1] Fig. 1 is a side view of a balloon catheter 10 according to a first embodiment.
[Fig. 2] Fig. 2 is a side view of the balloon catheter 10 in a deflated state.
[Fig. 3] Fig. 3 is a section view as seen from the direction of the arrows on a line I-I in Fig. 2.
[Fig. 4] Fig. 4 is a section view of the balloon catheter 10 in the deflated state.
[Fig. 5] Fig. 5 is a side view of the balloon catheter 10 in an inflated state.
[Fig. 6] Fig. 6 is a section view of the balloon catheter 10 in the inflated state.
[Fig. 7] Fig. 7 is a section view as seen from the direction of the arrows on a line II-II in Fig. 5.
[Fig. 8] Fig. 8 is a section view as seen from the direction of the arrows on the line II-II in Fig. 5.
[Fig. 9] Fig. 9 is a side view of a balloon catheter 20 according to a second embodiment.
[Fig. 10] Fig. 10 is a side view of a balloon catheter 30 according to a third embodiment.
[Fig. 11] Fig. 11 is a side view of a balloon catheter 40 according to a fourth embodiment.
[Fig. 12] Fig. 12 is a side view of a balloon catheter 50 according to a fifth embodiment.
[Fig. 13] Fig. 13 is a side view of a linear member 45 according to the fifth embodiment.
[Fig. 14] Fig. 14 is a section view of a balloon catheter 60 according to a sixth embodiment.
[Fig. 15] Fig. 15 is a section view as seen from the direction of the arrows on a line III-III in Fig. 14.
[Fig. 16] Fig. 16 is a flowchart showing a manufacturing method (1) for a balloon body 10A.
[Fig. 17] Fig. 17 is a flowchart showing a manufacturing method (2) for the balloon body 10A.
[Fig. 18] Fig. 18 is a flowchart showing a manufacturing method (3) for the balloon body 10A.
[Fig. 19] Fig. 19 is a flowchart showing a manufacturing method (4) for the balloon body 10A.

### Modes for Carrying Out the Invention

### First Embodiment (balloon catheter 10)

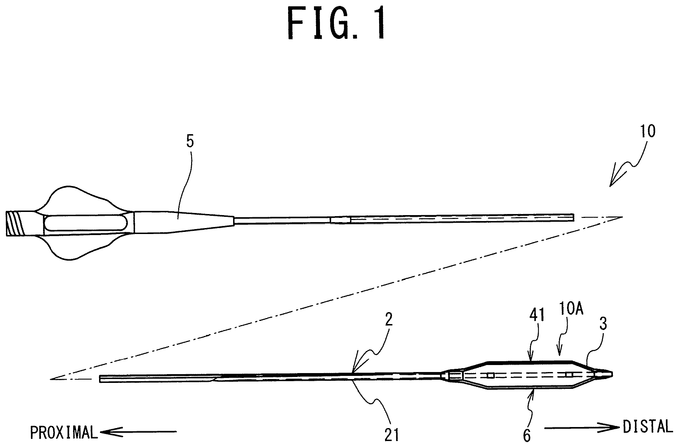

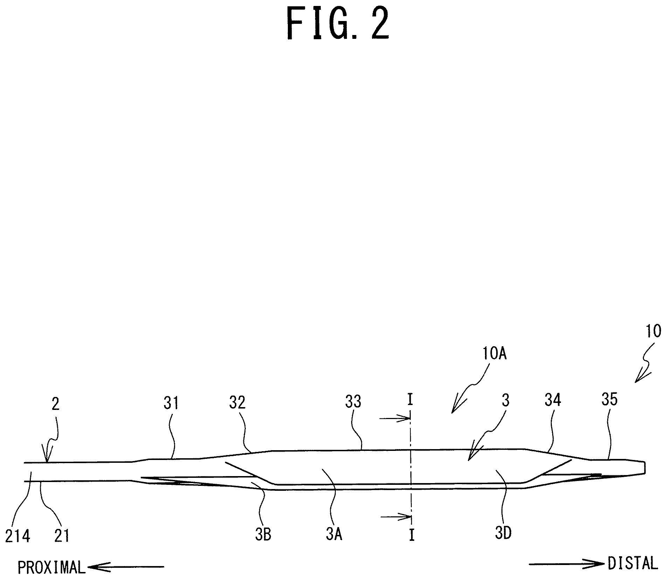

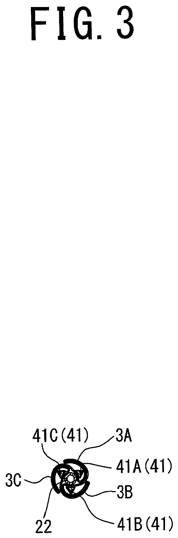

Hereinafter, a balloon catheter 10 according to a first embodiment of the present invention will be explained with reference to Figs. 1 to 8. As shown in Fig. 1, the balloon catheter 10 includes a catheter shaft 2, a balloon 3, linear members 41A, 41B, 41C (refer to Figs. 3 and the like; hereinafter collectively called the linear members 41), and a pressing member 6. The balloon 3 is connected to one end of the catheter shaft 2. Hereinafter, the end of the catheter shaft 2 to which the balloon 3 is connected will be called a distal end. The other end of the catheter shaft 2 will be called a proximal end. The linear members 41 are disposed on an outer circumferential surface 3D of the balloon 3 (refer to Fig. 2). The pressing member 6 covers the balloon 3 and the linear members 41 and presses the linear members 41 against the balloon 3. Hereinafter, the balloon 3, the linear members 41, and the pressing member 6 will be called a balloon body 10A.

The balloon catheter 10 is used in a state in which a hub 5 is connected to the proximal end of the catheter shaft 2. The hub 5 is capable of supplying a compressed fluid to the balloon 3 through the catheter shaft 2. A direction that extends along the catheter shaft 2 will be called a extension direction. On a plane that is orthogonal to the extension direction, in a radial direction with respect to the cross-sectional center of the catheter shaft 2, a side that is closer to the cross-sectional center of the catheter shaft 2 will be called an inner side, and a side that is farther from the cross-sectional center of the catheter shaft 2 will be called an outer side.

### Catheter shaft 2

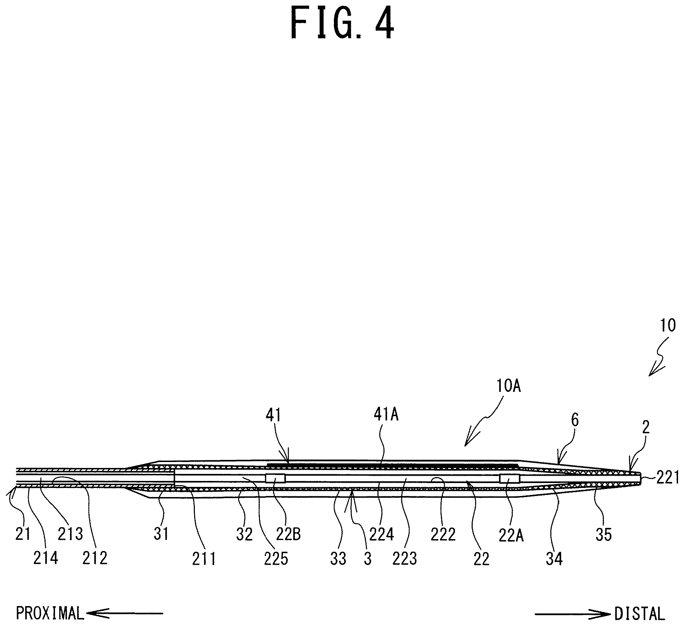

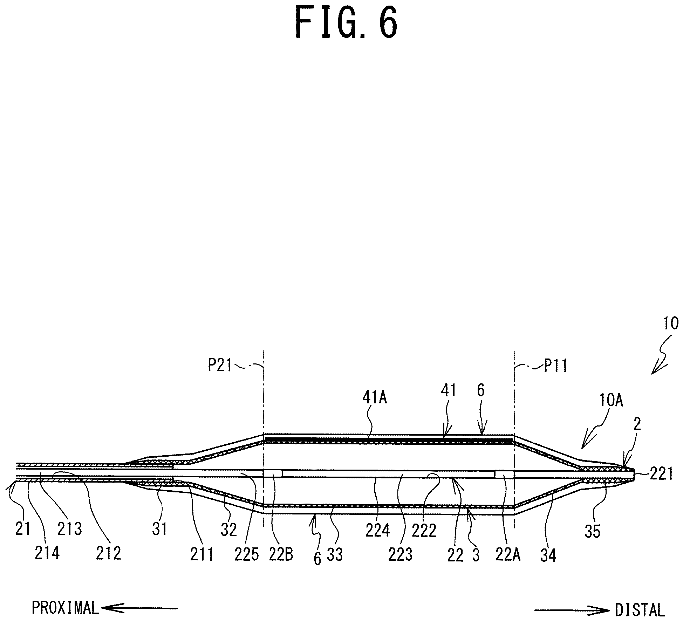

As shown in Figs. 4 and 6, the catheter shaft 2 includes an outer side tube 21 and an inner side tube 22. The outer side tube 21 and the inner side tube 21 are each flexible tubular members. The outer side tube 21 includes an inner cavity 213, which is a space that is enclosed by an inner face 212. The inner side tube 22 includes an inner cavity 223, which is a space that is enclosed by an inner face 222. The outer side tube 21 and the inner side tube 22 are formed from a polyamide resin. The inside diameter of the outer side tube 21 is larger than the outside diameter of the inner side tube 22.

The inner side tube 22 is disposed inside the inner cavity 213 of the outer side tube 21, except for a designated portion at the distal end of the inner side tube 22. The designated portion at the distal end of the inner side tube 22 protrudes toward the distal end of the catheter shaft 2 from the distal end of the outer side tube 21 (hereinafter called the distal end 211). Therefore, the distal end of the inner side tube 22 (hereinafter called the distal end 221) is disposed farther toward the distal end of the catheter shaft 2 than is the distal end 211 of the outer side tube 21. Hereinafter, the designated portion at the distal end of the inner side tube 22 will be called a protruding portion 225. Radiopaque markers (hereinafter simply called the markers) 22A, 22B are mounted on the protruding portion 225 of the inner side tube 22. A resin into which a radiopaque material is mixed is used as the material of the markers 22A, 22B. Hollow cylindrical members that are formed from the material are crimped onto the protruding portion 225 of the inner side tube 22 in order to affix the markers 22A, 22B to an outer circumferential surface 224 of the protruding portion 225 of the inner side tube 22. The markers 22A, 22B have specified lengths in the extension direction. The markers 22A, 22B do not allow the passage of radiation. The marker 22A is disposed closer to the distal end of the catheter shaft 2 than is the marker 22B. The markers 22A, 22B are set apart from one another in the extension direction.

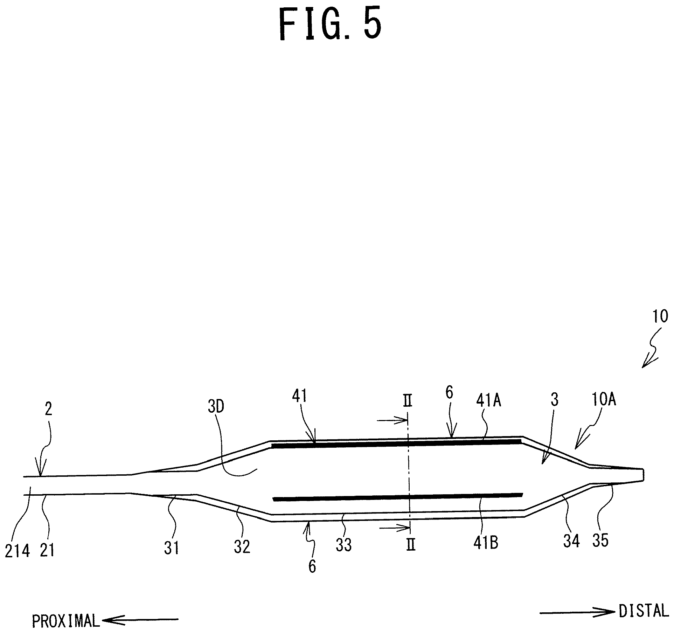

The compressed fluid that is supplied by the hub 5 (refer to Fig. 1) flows through the space that is inside the inner cavity 213 of the outer side tube 21 and outside the inner cavity 223 of the inner side tube 22. The balloon 3 inflates in response to the supplying of the compressed fluid (refer to Figs. 5 to 8). A guide wire that is not shown in the drawings is inserted into the inner cavity 223 of the inner side tube 22.

The material of the outer side tube 21 and the inner side tube 22 is not limited to being a polyamide resin, and it can be changed to another flexible material. For example, a synthetic resin material such as a polyethylene resin, a polypropylene resin, a polyurethane resin, a polyimide resin, or the like may also be used as the material of the outer side tube 21 and the inner side tube 22. An additive may also be mixed into the synthetic resin material. Different synthetic resin materials may also be used as the materials of the outer side tube 21 and the inner side tube 22. The material of the markers 22A, 22B is not limited to being a resin into which a radiopaque material has been mixed, and it can be changed to another material that does not allow the passage of radiation. For example, a resin that has been coated with a radiopaque material by vapor deposition, a material such as metal or the like that does not allow the passage of radiation, or the like may also be used as the material of the markers 22A, 22B.

### Balloon 3

As shown in Figs. 2 to 4, in a state in which the compressed fluid is not supplied, the balloon 3 contracts inward. In contrast, the balloon 3 expands outward in a state in which the compressed fluid has been supplied, as shown in Figs. 5 to 8. The balloon 3 is formed from a polyamide resin. As shown in Figs. 2 and 4 to 6, the balloon 3 includes a proximal end side leg portion 31, a proximal end side cone region 32, an inflation region 33, a distal end side cone region 34, and a distal end side leg portion 35. The proximal end side leg portion 31, the proximal end side cone region 32, the inflation region 33, the distal end side cone region 34, and the distal end side leg portion 35 respectively correspond to five sections of the balloon 3, divided along the extension direction. The length of the inflation region 33 in the extension direction is greater than the individual lengths of the proximal end side leg portion 31, the proximal end side cone region 32, the distal end side cone region 34, and the distal end side leg portion 35 in the extension direction.

As shown in Figs. 4 and 6, the proximal end side leg portion 31 is connected by thermal welding to an outer circumferential surface 214 of the outer side tube 21, at a point that is closer to the proximal end than is the distal end 211. The proximal end side cone region 32 is contiguous with the distal end of the proximal end side leg portion 31. The inflation region 33 is contiguous with the distal end of the proximal end side cone region 32. The distal end side cone region 34 is contiguous with the distal end of the inflation region 33. The distal end side leg portion 35 is contiguous with the distal end of the distal end side cone region 34. The distal end side leg portion 35 is connected by thermal welding to the outer circumferential surface 224 of the protruding portion 225 of the inner side tube 22, at a point that is closer to the proximal end than is the distal end 221. The proximal end side leg portion 31, the proximal end side cone region 32, the inflation region 33, the distal end side cone region 34, and the distal end side leg portion 35 are arrayed in that order from the proximal end toward the distal end. The proximal end side cone region 32, the inflation region 33, the distal end side cone region 34, and the distal end side leg portion 35 cover the protruding portion 225 of the inner side tube 22 from the outer side.

As shown in Figs. 2 to 4, the balloon 3 is a three-pleat balloon, in which three pleats are formed in the balloon 3 when the balloon 3 is in the deflated state. As shown in Fig. 3, in the deflated state, the balloon 3 folds up such that pleats 3A, 3B, 3C are formed. The pleats 3A, 3B, 3C are wound around the protruding portion 225 of the inner side tube 22. In this state, the pleat 3A covers the linear member 41A, which will be described later, from the outer side. The pleat 3B covers the linear member 41B, which will be described later, from the outer side. The pleat 3C covers the linear member 41C, which will be described later, from the outer side. The pleats 3A, 3B, 3C are also called flaps and wings.

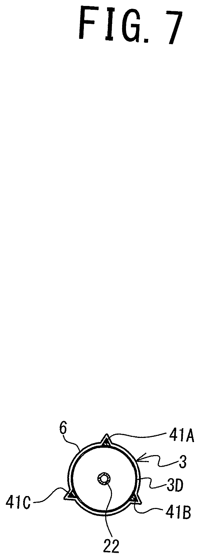

The balloon 3 in the inflated state will be explained with reference to Figs. 5 to 7. As shown in Fig. 7, the cross-sectional shape of the balloon 3 is circular. As shown in Figs. 5 and 6, the proximal end side cone region 32 has a tapered shape. The diameter of the proximal end side cone region 32 increases continuously and in a straight line from the proximal end toward the distal end. The inflation region 33 extends in the extension direction. The diameter of the inflation region 33 is substantially constant over its entire length in the extension direction. The distal end side cone region 34 has a tapered shape. The diameter of the distal end side cone region 34 decreases continuously and in a straight line from the proximal end toward the distal end. The cross-sectional diameter of the balloon 3 varies in stages through the proximal end side cone region 32, the inflation region 33, and the distal end side cone region 34. The inflation region 33 has the largest outside diameter of any part of the balloon 3.

As shown in Fig. 6, the boundary at the distal end of the inflation region 33, in other words, the position of the boundary between the inflation region 33 and the distal end side cone region 34, is coincident with a position P11, which is the position of the distal end of the marker 22A in the extension direction. The boundary at the proximal end of the inflation region 33, in other words, the position of the boundary between the inflation region 33 and the proximal end side cone region 32, is coincident with a position P21, which is the position of the proximal end of the marker 22B in the extension direction.

The material of the balloon 3 is not limited to being a polyamide resin, and it can be changed to another flexible material. For example, a polyethylene resin, a polypropylene resin, a polyurethane resin, a polyimide resin, silicone rubber, natural rubber, or the like may also be used as the material of the balloon 3. The method for connecting the outer side tube 21 and the inner side tube 22 to the balloon 3, and the method for connecting the outer side tube 21 to a mounting member 21A, are not limited to thermal welding. For example, each of the connections may also be made by using an adhesive.

### Linear members 41

The linear members 41 will be explained with reference to Figs. 4 to 8. The linear members 41 are monofilament elastic bodies that are resilient to bending deformation. The linear members 41A, 41B, 41C have identical shapes. The linear members 41 extend in the extension direction.

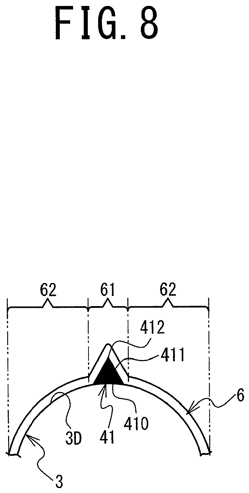

As shown in Figs. 4 to 7, the linear members 41 are disposed on the outer circumferential surface 3D of the inflation region 33 of the balloon 3 (refer to Figs. 5 and 7). The linear members 41 are pressed against the balloon 3 by the pressing member 6, which will be described later. The portion of each one of the linear members 41 that faces the balloon 3 (hereinafter called an inner side portion 410 (refer to Fig. 8)) is in contact with the outer circumferential surface 3D of the balloon 3. The linear members 41 are able to move in relation to the outer circumferential surface 3D of the balloon 3. The inner side portions 410 are not joined to the outer circumferential surface 3D of the balloon 3. The position of the proximal end of each one of the linear members 41 is substantially coincident with the position of the boundary between the inflation region 33 and the proximal end side cone region 32. The position of the distal end of each one of the linear members 41 is substantially coincident with the position of the boundary between the inflation region 33 and the distal end side cone region 34. The linear members 41 are not disposed on the outer circumferential surface 3D of the balloon 3 in the proximal end side leg portion 31, the proximal end side cone region 32, the distal end side cone region 34, and the distal end side leg portion 35.

As shown in Fig. 7, in the state in which the balloon 3 is inflated, the linear members 41A, 41B, 41C extend in straight lines in the extension direction at positions that are substantially equally spaced around the circumference of the balloon 3. As shown in Fig. 8, the cross-sectional shape of the linear member 41 is an equilateral triangle in which the inner side portion 410 is the bottom side. In the linear member 41, the most outward portion of an outer side portion 411, which is opposite the inner side portion 410, that is, an apex portion 412 of the equilateral triangle in Fig. 8, is pointed.

The linear members 41 are formed from a polyamide resin. The hardness of the linear members 41 is a value that is in the range of D70 to D95 in ISO868. The hardness of the linear members 41 is harder than the hardness of the balloon 3 over the entire range from the inner side portion 410 to the outer side portion 411. In this case, the linear members 41 are more resistant to expansion and contraction in the extension direction than is the balloon 3. In the present invention, the hardness of the balloon 3 may also be harder than that of the linear members 41. The linear members 41 may also expand and contract in the extension direction more readily than does the balloon 3.

### Pressing member 6

As shown in Figs. 4 to 8, the pressing member 6 is a membranous member that covers the entire balloon 3. The pressing member 6 covers the entire outer circumferential surface 3D of the balloon 3, or more specifically, the outer circumferential surface 3D of the proximal end side leg portion 31, the proximal end side cone region 32, the inflation region 33, the distal end side cone region 34, and the distal end side leg portion 35 of the balloon 3.

As shown in Fig. 8, the pressing member 6 includes first membrane portions 61 and second membrane portions 62. Each of the first membrane portions 61 is a portion of the pressing member 6 that is in contact with one of the linear members 41 and that directly covers the linear member 41 from the outer side. The linear member 41 is sandwiched between the first membrane portion 61 and the outer circumferential surface 3D of the balloon 3. The first membrane portion 61 adheres tightly to the outer side portion 411 of the linear member 41. Each of the second membrane portions 62 is a portion of the pressing member 6 that is in contact with the outer circumferential surface 3D of the balloon 3 and that directly covers the outer circumferential surface 3D from the outer side. Each of the second membrane portions 62 adheres tightly to the outer circumferential surface 3D of the balloon 3. Gaps are not formed between the first membrane portions 61 and the outer side portions 411 of the linear members 41 and between the second membrane portions 62 and the outer circumferential surface 3D of the balloon 3. The linear members 41 are pressed against the balloon 3 by the elasticity of the pressing member 6. Therefore, movement of the linear members 41 in relation to the balloon 3 is inhibited by the pressing member 6. Note that the force with which the pressing member 6 presses the linear members 41 against the balloon 3 needs only to be strong enough to prevent the linear members 41 from separating from the balloon 3 when the balloon 3 is in the inflated state. Therefore, the pressing member 6 needs only to be an elastic member that is positioned along the linear members 41 and the outer circumference of the inflated balloon 3, for example. Specifically, the pressing member 6 needs only to have a shape that conforms to the linear members 41 and the outer surface of the inflated balloon 3 when a state exists in which a force is not acting on the pressing member 6. In that case, the movement of the linear members 41 in relation to the inflated balloon 3 will be inhibited by the pressing member 6.

In a case where the balloon 3 inflates in response to the supplying of the compressed fluid from the hub 5, the inflation region 33 of the balloon 3 becomes longer in the extension direction. As explained previously, the inner side portions 410 of the linear members 41 are not joined to the balloon 3. The linear members 41 are more resistant to expansion and contraction in the extension direction than is the balloon 3. Therefore, in a case where the balloon 3 becomes longer, the inner side portions 410 of the linear members 41 slide in the extension direction in relation to the outer circumferential surface 3D of the balloon 3. The elongation of the inflation region 33 of the balloon 3 is not impeded by the linear members 41. The movement of the linear members 41 in the circumferential direction in relation to the outer circumferential surface 3D of the balloon 3 is inhibited by the pressing member 6. Therefore, when the balloon 3 is inflated, the movement of the linear members 41 in the circumferential direction is inhibited by the pressing member 6.

In contrast, in a case where the balloon 3 deflates in response to the discharging of the compressed fluid from the balloon 3, the inflation region 33 of the balloon 3, which had been elongated in the extension direction, becomes shorter due to its resilience. In this case, too, the inner side portions 410 of the linear members 41 slide in relation to the outer circumferential surface 3D of the balloon 3, in the same manner as they did when the balloon 3 was inflated. Therefore, the contraction of the inflation region 33 of the balloon 3 is not impeded by the linear members 41. The inflation region 33 of the balloon 3 contracts smoothly, such that the occurrence of wrinkles and the like in the balloon 3 is inhibited. The linear member 41A is covered from the outer side by the pleat 3A, the linear member 41B is covered from the outer side by the pleat 3B, and the linear member 41C is covered from the outer side by the pleat 3C (refer to Fig. 3). The material of the pressing member 6 may be a polyethylene resin, a polypropylene resin, a polyurethane resin, a polyimide resin, silicone rubber, natural rubber, or the like, for example. The thickness of the pressing member 6 may be from 5 to 40 µm, for example.

### Operation and effects of the first embodiment

As explained above, the balloon catheter 10 includes the pressing member 6, which presses the linear members 41 against the balloon 3. Therefore, when the balloon 3 is inflated, the balloon catheter 10 is better able to inhibit the linear members 41 from shifting position in relation to the balloon 3 than would be the case if the linear members 41 were attached to the balloon 3 by welding, adhesion, fusing, or the like. Because the linear members 41 are not directly attached to the balloon 3, they do not impede the lengthening of the outer circumferential surface 3D of the inflation region 33 when the balloon 3 is inflated. If the linear members 41 were to impede the inflating of the balloon 3, it is possible that the balloon 3 would bend in a direction that intersects the extension direction, which would not be desirable. In contrast to this, in the balloon catheter 10, the inflating of the balloon 3 is not readily inhibited by the linear members 41, so any bending of the balloon 3 when it is inflated can be prevented. The balloon catheter 10 is able to prevent the occurrence of wrinkles and the like in the balloon 3 when the balloon 3 is deflated.

The pressing member 6 covers the entire outer circumferential surface 3D of the proximal end side leg portion 31, the proximal end side cone region 32, the inflation region 33, the distal end side cone region 34, and the distal end side leg portion 35 of the balloon 3. The pressing member 6 adheres tightly to the balloon 3 and the linear members 41, without any gaps. Therefore, the pressing member 6 is able to press the entirety of each one of the linear members 41 against the balloon 3, so it is able to inhibit the linear members 41 from shifting position in relation to the balloon 3. Because the pressing member 6 is made in the form of a membrane, the balloon catheter 10 is able to use it to inhibit any enlarging of the diameter of the balloon body 10A. Accordingly, when the balloon body 10A moves through the interior of the blood vessel, the balloon catheter 10 is able to inhibit any diminishment of the balloon body 10A's ability to pass through the blood vessel due to snagging of the pressing member 6 on the inner wall of the blood vessel.

The linear members 41 are disposed on the inflation region 33 of the balloon 3. When the balloon 3 is inflated, the diameter of the inflation region 33 is substantially the same over its entire length in the extension direction. In this case, when the balloon 3 is inflated, the balloon catheter 30 is able to cause the linear members 41 on the inflation region 33 to act appropriately on the blood vessel. Because the hardness of the linear members 41 is harder than the hardness of the balloon 3, the balloon catheter 10 is able to cause the linear members 41 to act appropriately on the blood vessel when the balloon 3 is inflated. The outer side portions 411 of the linear members 41 are pointed at the apex portions 412. Therefore, when the balloon 3 is inflated, the outer side portions 411 readily dig into an injured portion of the blood vessel. Accordingly, by making it difficult for the balloon 3 to slide in relation to the injured portion of the blood vessel, the linear members 41 make it possible for the inflating of the balloon 3 to dilate the injured portion from the inside.

### Second Embodiment (balloon catheter 20)

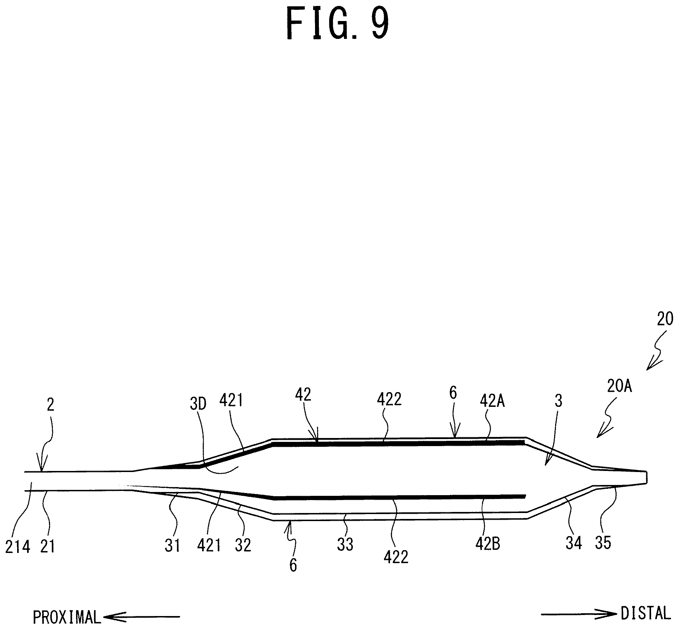

A balloon catheter 20 according to a second embodiment of the present invention will be explained with reference to Fig. 9. The balloon catheter 20 differs from the balloon catheter 10 according to the first embodiment (refer to Figs. 1 to 6 and the like) in that it is provided with linear members 42 instead of the linear members 41 (refer to Figs. 4 to 6 and the like). The linear members 42 include linear members 42A, 42B. The linear members 42A, 42B respectively correspond to the linear members 41A, 41B (refer to Figs. 7 and the like). The linear members 42 also include a linear member that is not shown in the drawings and that corresponds to the linear member 41C (refer to Fig. 7). A balloon body 20A corresponds to the balloon body 10A in the balloon catheter 10 (refer to Figs. 1 to 6 and the like).

As shown in Fig. 9, the linear members 42 are disposed on the outer circumferential surface 3D of the proximal end side leg portion 31, the proximal end side cone region 32, and the inflation region 33 of the balloon 3. Hereinafter, the portion of each one of the linear members 42 that is disposed on the outer circumferential surface 3D of the proximal end side leg portion 31 and the proximal end side cone region 32 will be called a proximal end portion 421, and the portion that is disposed on the outer circumferential surface 3D of the inflation region 33 will be called a inflation portion 422. The position of the proximal end of the proximal end portion 421 in the extension direction is substantially coincident with the proximal end of the proximal end side leg portion 31. The position of the distal end of the inflation portion 422 in the extension direction is substantially coincident with the position of the boundary between the inflation region 33 and the proximal end side cone region 32. The linear members 42 are not disposed on the outer circumferential surface 3D of the distal end side cone region 34 and the distal end side leg portion 35 of the balloon 3. The linear members 42 are entirely covered from the outer side by the pressing member 6.

### Operation and effects of the second embodiment

In the same manner as the balloon catheter 10, the balloon catheter 20 uses the pressing member 6 to inhibit the linear members 42 from shifting position in relation to the balloon 3 when the balloon 3 is inflated, and to inhibit the linear members 42 from interfering with the inflating of the balloon 3. The linear members 42 are longer than the linear members 41 in the extension direction. Therefore, in the balloon catheter 20, the surface area of contact between the linear members 42 and the balloon 3 and the pressing member 6 is greater than the corresponding surface area in the balloon catheter 10. Accordingly, the linear members 42 are pressed against the balloon 3 with greater force than are the linear members 41 of the balloon catheter 10. Accordingly, the balloon catheter 20 is able to inhibit the linear members 42 from shifting position in relation to the balloon 3 more reliably than the balloon catheter 10 can inhibit the shifting of the linear members 41.

In the balloon catheter 20, the linear members 42 are not disposed on the outer circumferential surface 3D of the balloon 3 in the distal end side cone region 34 and the distal end side leg portion 35. Therefore, the balloon catheter 20 is able to further reduce the diameter of the distal end portion of the balloon body 20A. Accordingly, a user is able to move the balloon body 20A of the balloon catheter 20 to a constricted location within a blood vessel using less force.

### Third Embodiment (balloon catheter 30)

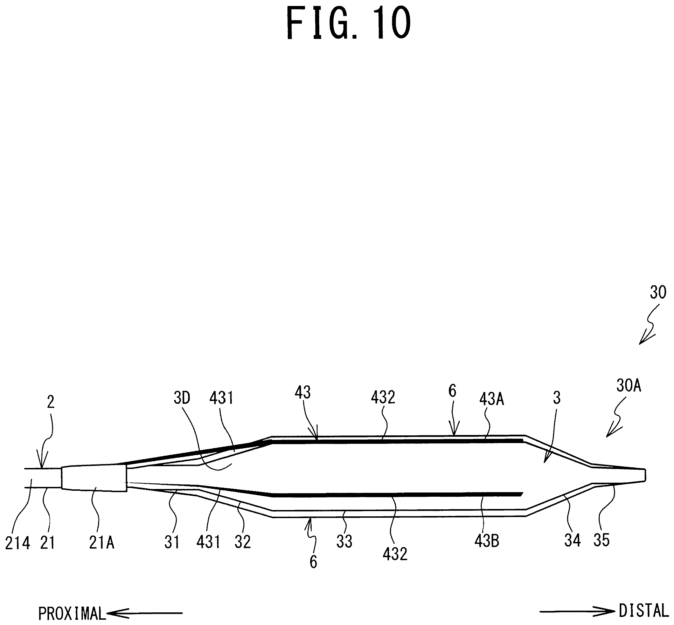

A balloon catheter 30 according to a third embodiment of the present invention will be explained with reference to Fig. 10. The balloon catheter 30 differs from the balloon catheter 20 according to the second embodiment (refer to Fig. 9) in that it is provided with linear members 43 instead of the linear members 42 (refer to Fig. 9) and is provided with the mounting member 21A. The linear members 43 differ from the linear members 42 in that portions of the linear members 43 are joined to the outer side tube 21 through the mounting member 21A. A balloon body 30A corresponds to the balloon body 20A in the balloon catheter 20 (refer to Fig. 9).

In the balloon catheter 30, the mounting member 21A is mounted on a portion of the outer circumferential surface 214 of the outer side tube 21 that is closer to the proximal end than is the distal end 211 (refer to Figs. 4 and 6). The mounting member 21A is a cylindrical member that is able to move along the extension direction. The inside diameter of the mounting member 21A is greater than the outside diameter of the outer side tube 21. A thermoplastic resin such as a polyamide resin or the like is used as the material of the mounting member 21A.

The linear members 43 include linear members 43A, 43B. The linear members 43A, 43B respectively correspond to the linear members 42A, 42B (refer to Fig. 9). The linear members 43 also include a linear member that is not shown in the drawings and that corresponds to the linear member 42 that is not shown in the drawings. Each one of the linear members 43 includes a proximal end portion 431 and an inflation portion 432. The proximal end portion 431 and the inflation portion 432 respectively correspond to the proximal end portion 421 and the inflation portion 422 of the linear member 42 (refer to Fig. 9). The proximal end of the proximal end portion 431 is connected to the outer circumferential surface of the mounting member 21A by thermal welding. Therefore, the linear member 43 is joined to the catheter shaft 2 (the outer side tube 21) through the mounting member 21A. The linear members 43 are not connected to the balloon 3 other than at the proximal ends of the proximal end portions 431.

The inflation portions 432 of the linear members 43 are in contact with the outer circumferential surface 3D of the inflation region 33 of the balloon 3. The proximal end portions 431 of the linear members 43 extend in straight lines toward the mounting member 21A from the boundary between the proximal end side cone region 32 and the inflation region 33 of the balloon 3. Therefore, the proximal end portions 431 are not in contact with the outer circumferential surface 3D of the proximal end side leg portion 31 and the proximal end side cone region 32 of the balloon 3 and are each set apart from the outer circumferential surface 3D to the outer side.

The pressing member 6 covers the entire outer circumferential surface 3D of the balloon 3. The inflation portions 432 of the linear members 43 are sandwiched between the pressing member 6 and the outer circumferential surface 3D of the balloon 3. The inflation portions 432 are pressed toward the balloon 3 by the elasticity of the pressing member 6. Movement of the linear members 43 in relation to the balloon 3 is thus inhibited by the pressing member 6. In contrast, the proximal end portions 431 of the linear members 43 are exposed to the outer side in relation to the pressing member 6.

### Operation and effects of the third embodiment

In the same manner as the balloon catheters 10, 20, the balloon catheter 30 uses the pressing member 6 to inhibit the linear members 43 from shifting position in relation to the balloon 3 when the balloon 3 is inflated, and to inhibit the linear members 43 from interfering with the inflating of the balloon 3. In the case of the balloon catheter 30, the proximal ends of the linear members 43 are joined to the catheter shaft 2 through the mounting member 21A. Therefore, in a case where a force in the direction of the distal end acts on the linear members 43 when the balloon 3 is withdrawn from within the blood vessel, the balloon catheter 30 can effectively inhibit the linear members 43 from shifting position in relation to the catheter shaft 2.

### Fourth Embodiment (balloon catheter 40)

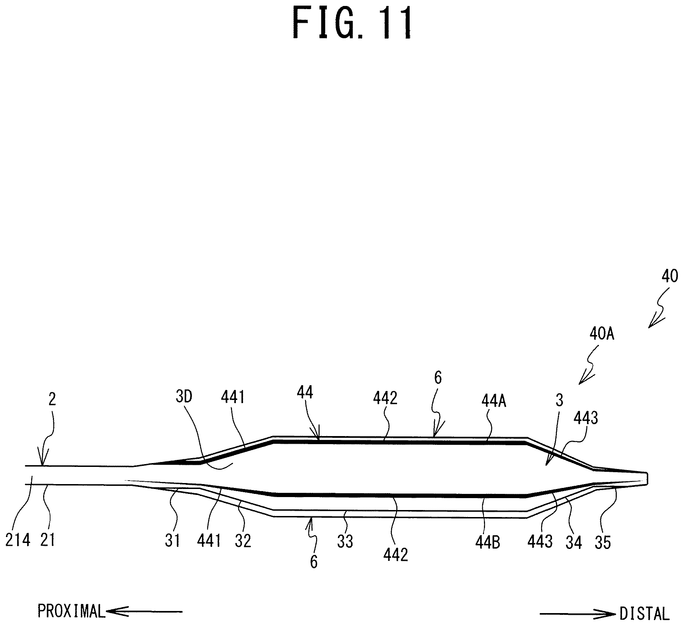

A balloon catheter 40 according to a fourth embodiment of the present invention will be explained with reference to Fig. 11. The balloon catheter 40 differs from the balloon catheter 10 according to the first embodiment (refer to Figs. 1 to 6 and the like) in that it is provided with linear members 44 instead of the linear members 41 (refer to Figs. 4 to 6 and the like). The linear members 44 include linear members 44A, 44B. The linear members 44A, 44B respectively correspond to the linear members 41A, 41B (refer to Figs. 7 and the like). The linear members 44 also include a linear member that is not shown in the drawings and that corresponds to the linear member 41C (refer to Fig. 7). A balloon body 40A corresponds to the balloon body 10A in the balloon catheter 10 (refer to Figs. 1 to 6 and the like).

As shown in Fig. 11, the linear members 44 are disposed on the outer circumferential surface 3D of the balloon 3 over the entire length of the extension direction. More specifically, the linear members 44 are disposed on the outer circumferential surface 3D of the proximal end side leg portion 31, the proximal end side cone region 32, the inflation region 33, the distal end side cone region 34, and the distal end side leg portion 35 of the balloon 3. Hereinafter, for each one of the linear members 44, the portion that is positioned on the outer circumferential surface 3D of the proximal end side leg portion 31 and the proximal end side cone region 32 will be called the proximal end portion 441, the portion that is positioned on the outer circumferential surface 3D of the inflation region 33 will be called the inflation portion 442, and the portion that is positioned on the outer circumferential surface 3D of the distal end side cone region 34 and the distal end side leg portion 35 will be called a distal end portion 443. The proximal end portions 441 and the inflation portions 442 respectively correspond to the proximal end portions 421 and the inflation portions 422 of the linear members 42 (refer to Fig. 9). In the extension direction, the position of the distal end of the distal end portion 443 is substantially coincident with the position of the distal end of the distal end side leg portion 35. The linear members 44 are entirely covered from the outer side by the pressing member 6.

### Operation and effects of the fourth embodiment

In the same manner as the balloon catheters 10, 20, 30, the balloon catheter 40 uses the pressing member 6 to inhibit the linear members 44 from shifting position in relation to the balloon 3 when the balloon 3 is inflated, and to inhibit the linear members 44 from interfering with the inflating of the balloon 3. The linear members 44 are longer than the linear members 41, 42, 43 in the extension direction. Therefore, in the balloon catheter 40, the surface area of contact between the linear members 44 and the balloon 3 and the pressing member 6 is greater than the corresponding surface area in any one of the balloon catheters 10, 20, 30. Accordingly, the linear members 44 are pressed against the balloon 3 with greater force than are the linear members 41, 42, 43. Accordingly, the balloon catheter 40 is able to inhibit the linear members 44 from shifting position in relation to the balloon 3 more reliably than the balloon catheters 10, 20, 30 can inhibit the shifting of the linear members 41, 42, 43.

### Fifth Embodiment (balloon catheter 50)

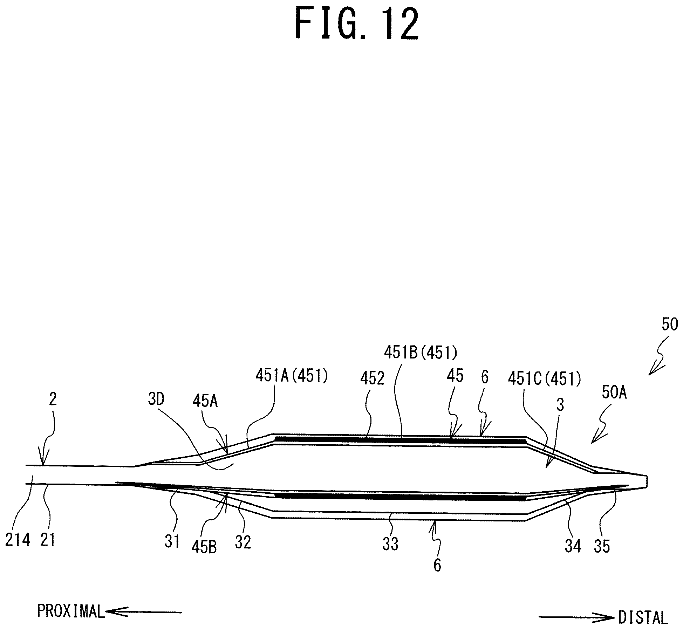

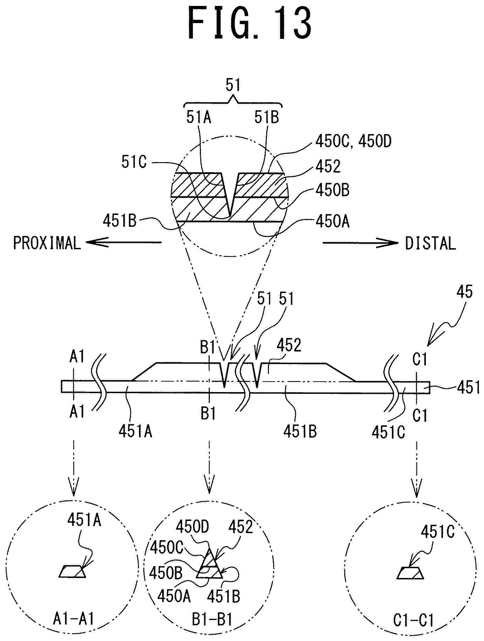

A balloon catheter 50 according to a fifth embodiment of the present invention will be explained with reference to Figs. 12 and 13. The balloon catheter 50 differs from the balloon catheter 10 according to the first embodiment (refer to Figs. 1 to 6 and the like) in that it is provided with linear members 45 instead of the linear members 41 (refer to Figs. 4 to 6 and the like). The linear members 45 include linear members 45A, 45B. The linear members 45A, 45B respectively correspond to the linear members 41A, 41B (refer to Figs. 7 and the like). The linear members 45 also include a linear member that is not shown in the drawings and that corresponds to the linear member 41C (refer to Fig. 7). A balloon body 50A corresponds to the balloon body 10A in the balloon catheter 10 (refer to Figs. 1 to 6 and the like).

As shown in Fig. 12, each one of the linear members 45 includes a soft portion 451 and a hard portion 452. The soft portion 451 extends all the way from the proximal end of the proximal end side leg portion 31 to the distal end of the distal end side leg portion 35. The soft portion 451 includes a first portion 451A, a second portion 451B, and a third portion 451C. The first portion 451A, the second portion 451B, and the third portion 451C are each one of three portions into which the soft portion 451 is divided along the extension direction. The first portion 451A is disposed on the outer circumferential surface 3D of the proximal end side leg portion 31 and the proximal end side cone region 32 of the balloon 3. The second portion 451B is disposed on the outer circumferential surface 3D of the inflation region 33 of the balloon 3. The third portion 451C is disposed on the outer circumferential surface 3D of the distal end side cone region 34 and the distal end side leg portion 35 of the balloon 3. The hard portion 452 is laminated onto the second portion 451B of the soft portion 451 on the opposite side of the second portion 451B from the side that faces the balloon 3. The first portion 451A and the third portion 451C of the soft portion 451 respectively correspond to the proximal end portion 441 and the distal end portion 443 of the linear member 44 in the balloon catheter 40 (refer to Fig. 11). The hard portion 452 and the second portion 451B of the soft portion 451 correspond to the inflation portion 442 of the linear member 44 (refer to Fig. 11).

Fig. 13 shows cross sections of the linear member 45 at a line A1-A1, a line B1-B1, and a line C1-C1 in a state in which a force in the extension direction is not acting on the linear member 45. The cross-sectional shape of the soft portion 451 (the first portion 451A to the third portion 451C) is a trapezoid. The cross-sectional shape of the hard portion 452 is an equilateral triangle, one side of which is formed from a portion of the second portion 451B of the soft portion 451 that faces the balloon 3 (hereinafter called the inner side portion 450A) and a portion on the opposite side of the second portion 451B (hereinafter called a boundary portion 450B). The hard portion 452 protrudes to the outer side from the boundary portion 450B. Hereinafter, the portion of the hard portion 452 on the outer side will be called the outer side portion 450C. Within the outer side portion 450C, the point that corresponds to the apex of the equilateral triangle will be called an apex 450D. The apex 450D is pointed.

The linear members 45 are formed from a polyamide resin. More specifically, the soft portion 451 is formed from a polyamide elastomer. The hardness of the soft portion 451 is a value that is in the range of D25 to D63 in ISO868. The hard portion 452 is formed from a polyamide resin. The hardness of the hard portion 452 is a value that is in the range of D70 to D95 in ISO868. That is, the hardness of the hard portion 452 is harder than the hardness of the soft portion 451. The soft portion 451 can be stretched more than can the hard portion 452.

Two notches 51 are formed in each one of the linear members 45, extending radially inward from the outer side portion 450C of the hard portion 452. Each one of the two notches 51 is formed by removing a portion of the linear member 45. The cross-sectional shape of each of the notches 51 is a wedge shape. The two notches 51 are arrayed at a specified interval in the extension direction. Each one of the notches 51 includes faces 51A, 51B, which are opposite one another in the extension direction. An end portion (hereinafter called a bottom portion) 51C at the inner end of each one of the notches 51 is positioned farther inward radially from the boundary portion 450B between the hard portion 452 and the second portion 451B of the soft portion 451.

When the balloon 3 is inflated in response to the supplying of the compressed fluid from the hub 5, the balloon 3 becomes longer in the extension direction. Therefore, a force acts in the extension direction on the soft portions 451 of the linear members 45, which are in contact with the outer circumferential surface 3D of the balloon 3. In this case, the first portions 451A and the third portions 451C are deformed elastically such that they become elongated in the extension direction. The force in the extension direction also acts on the portions of the linear members 45 where the hard portions 452 are laminated onto the second portions 451B12 of the soft portions 451. Here, the hard portions 452 do not become elongated as readily as the soft portions 451 do. In response to the elastic deforming of the second portions 451B of the soft portions 451 by the force in the extension direction, the faces 51A, 51B in the plurality of the notches 51 move farther apart from one another in the extension direction. Therefore, the elastic deformation of the second portions 451B of the soft portions 451 is not inhibited by the hard portions 452. Accordingly, even the portions of the linear members 45 where the hard portions 452 are laminated onto the second portions 451B of the soft portions 451 are elastically deformed such that they become elongated in the extension direction in response to the inflating of the balloon 3. The linear members 45 thus become elongated over their entire lengths in the extension direction in accordance with the inflating of the balloon 3.

### Operation and effects of the fifth embodiment

It is possible for the soft portions 451 of the linear members 45 to become elongated. Therefore, in a case where the linear members 45 become elongated in response to the inflating of the balloon 3, the first portions 451A and the third portions 451C of the soft portions 451, onto which the hard portions 452 are not laminated, readily become elongated along with the balloon 3. Two of the notches 51 are formed in each one of the linear members 45. Therefore, in a case where the second portions 451B of the soft portions 451 become elongated in response to the inflating of the balloon 3, the faces 51A, 51B in each one of the notches 51 move farther apart from one another. The hard portions 452 are thus inhibited from interfering with the elongation of the second portions 451B of the soft portions 451. Therefore, the linear members 45 can be elongated in conjunction with the inflating of the balloon 3 by becoming appropriately elongated over their entire lengths in response to the inflating of the balloon 3. Accordingly, the balloon catheter 50 is able to inhibit the linear members 45 from interfering with the inflating of the balloon 3.

When the balloon 3 is inflated, the outer side portions 450C of the hard portions 452 of the linear members 45 protrude toward the outer side in relation to the balloon 3. The hardness of the hard portions 452 is harder than the hardness of the soft portions 451. Therefore, when the balloon 3 is inflated, the linear members 45 are able to cause the hard portions 452 to act appropriately on the blood vessel.

In the balloon catheter 50, the faces 51A, 51B in the plurality of the notches 51 move farther apart from one another in the extension direction when the balloon 3 is inflated. This enables the second portions 451B of the soft portions 451 to readily be elastically deformed. However, in some cases, the forming of the plurality of the notches 51 in each one of the linear members 45 diminishes the strength of the linear members 45 themselves in the areas where the plurality of the notches 51 are located. In the fifth embodiment, this problem is addressed by using the pressing member 6 to entirely cover the linear members 45 from the outer side. Therefore, even in a case where the reduced strength of the linear member 45 causes the linear member 45 to break at the locations of the notches 51, the linear member 45 is not detached from the balloon 3. Thus, in a case where the linear member 45 has broken at the locations of the notches 51, the pressing member 6 can inhibit the linear member 45 from being detached from the balloon 3.

### Sixth Embodiment (balloon catheter 60)

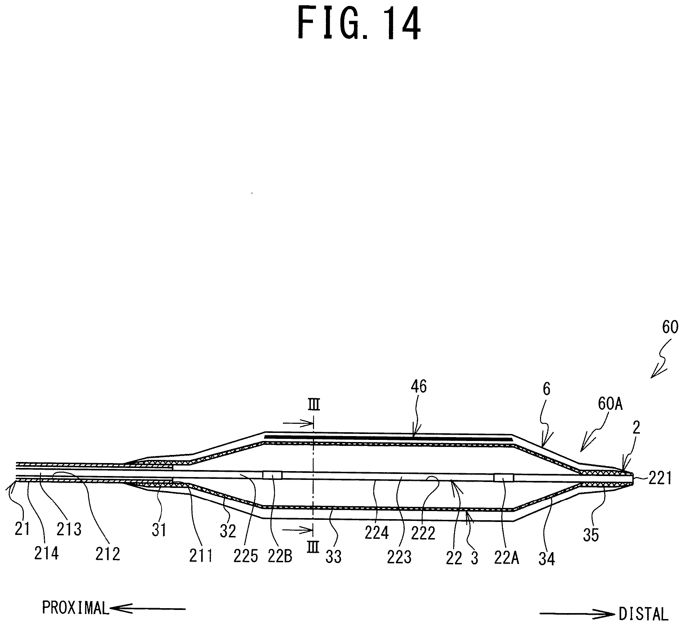

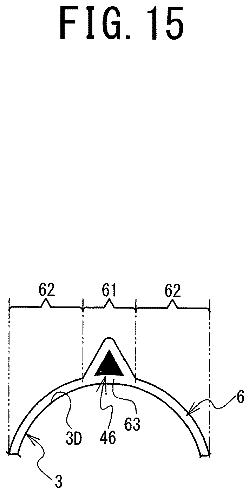

A balloon catheter 60 according to a sixth embodiment of the present invention will be explained with reference to Figs. 14 and 15. Linear members 46 of the balloon catheter 60 in the sixth embodiment have the same shape as the linear members 41 (refer to Figs. 4 to 6 and the like) of the balloon catheter 10 (refer to Figs. 1 to 6 and the like). As shown in Fig. 15, the balloon catheter 60 differs from the balloon catheter 10 in that portions of the pressing member 6 are disposed between the balloon 3 and the outer circumferential surface 3D of the linear members 46. Hereinafter, the portions of the pressing member 6 that are disposed between the linear members 46 and the outer circumferential surface 3D of the balloon 3 will be called joining portions 63. A balloon body 60A corresponds to the balloon body 10A of the balloon catheter 10 (refer to Figs. 1 to 6 and the like).

The pressing member 6 includes the first membrane portions 61, the second membrane portions 62, and the joining portions 63. In the manufacturing process for the balloon catheter 60, when the pressing member 6 is formed, the joining portions 63 penetrate between the linear members 46 and the outer circumferential surface 3D of the balloon 3 such that they join the linear members 46 to the outer circumferential surface 3D of the balloon 3. In the pressing member 6, at the same time that the first membrane portions 61 and the second membrane portions 62 press the linear members 46 against the balloon 3, the joining portions 63 join the linear members 46 to the balloon 3. A coating material that makes up the pressing member 6 penetrates between the linear members 46 and the outer circumferential surface 3D of the balloon 3. Drying the material creates a state in which the balloon 3 and the linear members 46 adhere to one another. The joining portions 63 are thus formed. Providing the joining portions 63 increases the friction between the balloon 3 and the linear members 46. The linear members 46 become elongated in response to the inflating of the balloon 3.

### Operation and effects of the sixth embodiment

In the balloon catheter 60, in addition to the pressing of the linear members 46 against the balloon 3 by the first membrane portions 61 and the second membrane portions 62 of the pressing member 6, the linear members 46 are joined to the outer circumferential surface 3D of the balloon 3 by the joining portions 63 of the pressing member 6. Therefore, the balloon catheter 60 is able to inhibit the linear members 46 from shifting position in relation to the balloon 3 even more appropriately. By using the joining portions 63 to join the linear members 46 to the outer circumferential surface 3D of the balloon 3, the balloon catheter 60 is able to prevent the positions of the linear members 46 from shifting in relation to the balloon 3. Therefore, in a state in which the linear members 46 are held in appropriate positions in relation to balloon 3, the balloon catheter 60 is able to cause the linear members 46 to act on the blood vessel when the balloon 3 is inflated.

### Seventh Embodiment (manufacturing method for balloon body 10A)

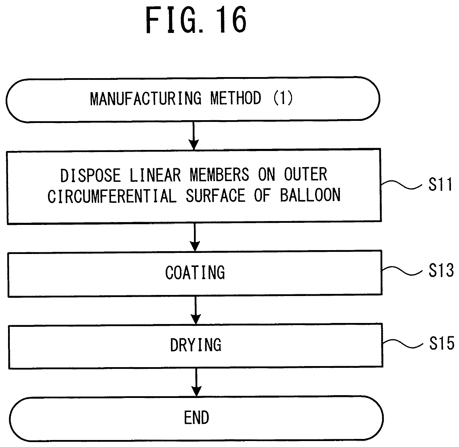

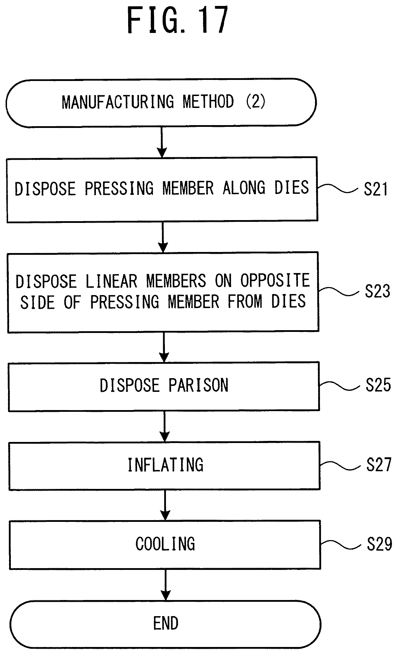

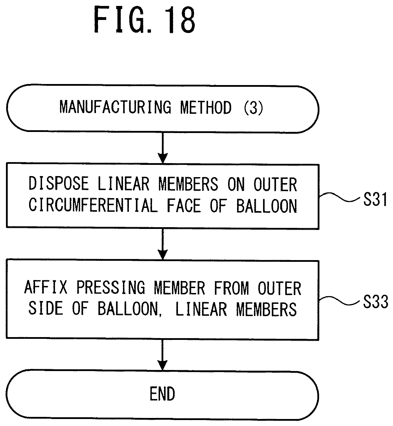

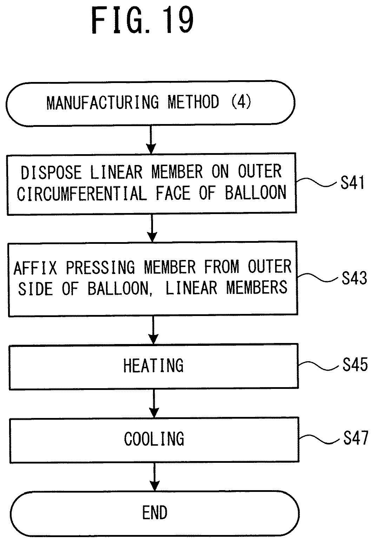

Specific examples of manufacturing methods for the balloon body 10A (refer to Figs. 1 to 6 and the like) will be explained with reference to Figs. 16 to 19. The balloon body 10A can be manufactured by methods of (1) applying a coating (refer to Fig. 16), (2) blow molding (refer to Fig. 17), (3) affixing the pressing member 6 (refer to Fig. 18), and (4) utilizing a heat shrinking property of the pressing member 6 (refer to Fig. 19). The explanation that follows uses the manufacturing of the balloon body 10A according to the first embodiment as an example, but the balloon bodies 20A (refer to Fig. 9), 30A (refer to Fig. 10), 40A (refer to Fig. 11), 50A (refer to Fig. 12), and 60A (refer to Fig. 14) according to the second to the sixth embodiments can also be manufactured by the same methods. The manufacturing methods (1) to (4) above are merely illustrative examples of the manufacturing methods for the balloon body 10A, and it goes without saying that the balloon body 10A can also be manufactured using manufacturing methods other than the methods (1) to (4).

### (1) Applying a coating

The method for manufacturing the balloon body 10A by applying a coating will be explained with reference to Fig. 16. This manufacturing method is based on a coating method that is known as a method for forming a thin film. Details of the method will be explained below.

The linear members 41 are disposed on the portion of the cylindrical balloon 3 that corresponds to the outer circumferential surface 3D of the inflation region 33 (Step S11). At this time, an adhesive or the like may be used in order to temporarily maintain the positional relationships between the balloon 3 and the linear members 41. Next, a spray is used to apply a molten coating to the balloon 3, on the outer circumferential surface 3D of which the linear members 41 are disposed (Step S13). The length of time that the coating is applied is optimized in accordance with the film thickness of the pressing member 6.

After the application of the coating has been completed, surface tension causes the molten coating to enter a state in which it covers the balloon 3 and the linear members 41 from the outer side. In some cases, the molten coating not only covers the surfaces of the balloon 3 and the linear members 41 from the outer side, but also penetrates into gaps between the balloon 3 and the linear members 41.

Next, the balloon 3 and the linear members 41 to which the molten coating has been applied using a spray are dried (Step S15). The temperature at this time is adjusted in accordance with the properties of the molten coating. The drying causes the molten coating that covers the balloon 3 and the linear members 41 from the outer side to form the first membrane portions 61 and the second membrane portions 62 of the pressing member 6. The first membrane portions 61 and the second membrane portions 62 of the pressing member 6 adhere tightly to the linear members 41 and the balloon 3, and they press the linear members 41 against the outer circumferential surface 3D of the balloon 3. The drying also causes the molten coating that has penetrated into the gaps between the balloon 3 and the linear members 41 to form the joining portions 63 of the pressing member 6. The joining portions 63 of the pressing member 6 join the linear members 41 to the balloon 3.

In the balloon body 10A that has been manufactured as described above, the linear members 41 are pressed firmly against the outer circumferential surface 3D of the balloon 3 by the first membrane portions 61 and the second membrane portions 62 of the pressing member 6. In a case where the joining portions 63 have been formed, the linear members 41 are directly joined to the outer circumferential surface 3D of the balloon 3 by the joining portions 63. Therefore, movement of the linear members 41 in relation to the balloon 3 is appropriately inhibited by the pressing member 6. The use of a spray to apply a coating is generally known as a method that can form, comparatively easily, a thin film that adheres tightly to an uneven surface. Therefore, the balloon body 10A, in which the linear members 41 do not readily shift position in relation to the outer circumferential surface 3D of the balloon 3, can be easily manufactured by the manufacturing method (1).

In the manufacturing method (1), the method of applying the molten coating is not limited to using a spray to apply the coating, and other known methods may also be used. For example, the molten coating may also be applied to the balloon 3 and the linear members 41 based on a dip coating method. The molten coating may also be applied to the balloon 3 and the linear members 41 using a brush or the like, for example.

### (2) Blow molding

The method for manufacturing the balloon body 10A by blow molding will be explained with reference to Fig. 17. This manufacturing method is based on a blow molding method that is known as a method for manufacturing a hollow molded item. Details of the method will be explained below.

A set of (male and female) dies for blow molding are prepared, the dies having inner walls that have the same shape as the inflated balloon 3. The membranous pressing member 6 is disposed along the inner walls of the set of the dies (Step S21). Next, the linear members 41 are disposed along the surface of the pressing member 6 that is on the opposite side of the pressing member 6 from the surface that faces the inner walls of the set of the dies (Step S23). At this time, an adhesive or the like may be used in order to temporarily maintain the positional relationships between the balloon 3 and the linear members 41. Next, the set of the dies is assembled. Next, a parison is poured into the interior of the assembled dies (Step S25). The parsion is a tubular piece of material that forms the basis for the balloon 3, and it is made from the raw material for the balloon 3. The parison is poured into the dies on the opposite side of the linear members 41 from the pressing member 6. The linear members 41 are sandwiched between the parison and the pressing member 6.

Next, air is blown into the interior of the parison (Step S27). The parison is inflated by the blowing in of the air. The parison comes into contact with the linear members 41 and the pressing member 6 from the inner side and is pressed against the inner walls of the dies. The balloon 3 is thus formed such that it has the same shape as the inner walls of the dies. The linear members 41 and the pressing member 6 adhere tightly to the outer circumferential surface 3D of the balloon 3. The linear members 41 are sandwiched between the balloon 3 and the pressing member 6.

The dies are opened, and the balloon 3, the linear members 41 and the pressing member 6 are removed. The balloon 3, the linear members 41 and the pressing member 6 that have been removed are cooled (Step S29). The balloon body 10A is thus formed. The pressing member 6 presses the linear members 41 against the outer circumferential surface 3D of the balloon 3.

In the manufacturing method (2), the linear members 41 are pressed firmly against the outer circumferential surface 3D of the balloon 3 by the pressing member 6, so the pressing member 6 can cause the linear members 41 to adhere tightly to the balloon 3. In the manufacturing method (2), unlike in the manufacturing method (1), the balloon body 10A can be manufactured using the pressing member 6 in a solid condition, without having to liquefy it or otherwise modify it. Therefore, it is possible to inhibit any changes in the physical properties of the pressing member 6 that might be due to changes in the state of the pressing member 6 at the time of manufacturing.

### (3) Affixing the pressing member 6

The method for manufacturing the balloon body 10A by affixing the pressing member 6 to the balloon 3 will be explained with reference to Fig. 18. The linear members 41 are disposed on the outer circumferential surface 3D of the balloon 3 (Step S31). Next, the membranous pressing member 6 is prepared. An adhesive is applied to one surface of the pressing member 6 or to the outer circumferential surface 3D of the balloon 3. The pressing member 6 is affixed to the outer circumferential surface 3D of the balloon 3 by the adhesive (Step S33). The outer circumferential surface 3D of the balloon 3 and the outer side surfaces of the linear members 41 are thus covered by the pressing member 6 from the outer side. The linear members 41 are pressed against the balloon 3 by the pressing member 6. The linear members 41 are sandwiched between the balloon 3 and the pressing member 6. The manufacturing method (3) eliminates the need for large-scale equipment in order to manufacture the balloon body 10A. Therefore, the balloon body 10A can be manufactured more easily than is possible with the manufacturing methods (1) and (2). In the process at Step S31, an adhesive may be used in order to temporarily maintain the positional relationships between the balloon 3 and the linear members 41. In that case, the linear members 41 can be affixed to the balloon 3 using the same adhesive that is used to affix the pressing member 6 to the balloon 3 and the linear members 41, for example. In that case, the outer circumferential surface 3D of the balloon 3, the pressing member 6, and the linear members 41 can be joined using an ordinary adhesive.

### (4) Method that utilizes the heat shrinking property of the pressing member 6

The method for manufacturing the balloon body 10A by utilizing the heat shrinking property of the pressing member 6 will be explained with reference to Fig. 19. In this case, it is presumed that the pressing member 6 has a property (heat shrinking) of shrinking in response to heating. Details of the method will be explained below.

The linear members 41 are disposed on the outer circumferential surface 3D of the balloon 3 (Step S41). Next, the membranous pressing member 6, which has the heat shrinking property, is prepared. Next, the outer side surfaces of the linear members 41 and the outer circumferential surface 3D of the balloon 3 are covered from the outer side by the pressing member 6 (Step S43). In this state, the pressing member 6 is heated (Step S45). The pressing member 6 is shrunk toward the inner side by the heating. The pressing member 6 adheres tightly to the linear members 41 and the outer circumferential surface 3D of the balloon 3 from the outer side. The linear members 41 are pressed against the balloon 3 by the pressing member 6. Next, the heating of the pressing member 6 is halted, and the pressing member 6 is cooled (Step S47). The linear members 41 are sandwiched between the balloon 3 and the pressing member 6.

In the manufacturing method (4), the linear members 41 are pressed firmly against the outer circumferential surface 3D of the balloon 3 by the pressing member 6, so the pressing member 6 can cause the linear members 41 to adhere tightly to the balloon 3. In the same manner as the manufacturing method (3), the manufacturing method (4) is a dry manufacturing method, so it can manufacture the balloon body 10A easily without requiring large-scale equipment. Unlike the manufacturing method (3), the manufacturing method (4) does not require an adhesive for affixing the pressing member 6. Therefore, the manufacturing method (4) can manufacture the balloon body 10A even more easily than can the manufacturing method (3).

### Modified examples

The present invention is not limited to the embodiments that are described above, and various types of modifications can be made. It is acceptable for the catheter shaft 2 not to include the outer side tube 21 and the inner side tube 22. For example, the catheter shaft 2 may also include only a single flexible tube. The numbers of the linear members 41 to 46 are not limited to being three, and other quantities may also be used. The cross-sectional shapes of the linear members 41 to 46 do not have to be equilateral triangles, and they may also be isosceles triangles and triangles having three sides of different lengths. The linear members 41 to 46 may also function as cutting edges that make incisions in the injured portion of the blood vessel when the balloon 3 is in the inflated state, for example.

In the inflated balloon 3, the boundary portion between the proximal end side cone region 32 and the inflation region 33, as well as the boundary portion between the inflation region 33 and the distal end side cone region 34, may be curved. In the embodiments that are described above, the respective diameters of the proximal end side cone region 32 and the distal end side cone region 34 change in straight lines from the proximal ends to the distal ends of the regions. However, it is also acceptable for the respective diameters of the proximal end side cone region 32 and the distal end side cone region 34 change in curved lines from the proximal ends to the distal ends of the regions. It is also acceptable for the diameter of one of the proximal end side cone region 32 and the distal end side cone region 34 to change in a curved line, and for the diameter of the other one of the proximal end side cone region 32 and the distal end side cone region 34 to change in a straight line. It is also acceptable for the markers 22A, 22B not to be provided on the inner side tube 22.

The linear members 41 to 46 may also extend in helical form in the extension direction. One of some and all of the portion of any one of the linear members 41 to 45 that faces the outer circumferential surface 3D of the inflation region 33 of the balloon 3 may also be joined to the outer circumferential surface 3D of the balloon 3. The method of the joining in that case is not limited, and may be, for example, any one of joining by an adhesive, welding, fusing, and the like. It is also acceptable for the pressing member 6 to cover only a portion of the outer circumferential surface 3D of the balloon 3. For example, the pressing member 6 may cover only the outer circumferential surface 3D of the inflation region 33 of the balloon 3. The pressing member 6 may also cover only those portions of the outer circumferential surface 3D of the inflation region 33 of the balloon 3 that are close to the linear members 41 to 46, for example. In that case, portions of the linear members 41 to 46 are exposed, instead of being covered by the pressing member 6. Furthermore, in a case where the pressing member 6 covers only the portions that are close to the linear members 41 to 46, the shape of the pressing member 6 is not limited to being a membrane, for example. Also, in a state in which a force is not acting on the pressing member 6, the inside diameter of the pressing member 6 may also be smaller than the outside diameter of the inflated balloon 3. In that case, the elastic force of the pressing member 6 acts in the direction that causes the inflated balloon 3 to contract, so the pressing member 6 presses the linear members 41 against the balloon 3.

The linear members 41 to 46 may also be disposed only on the outer circumferential surface 3D of the portions of the balloon 3 other than the inflation region 33 (the proximal end side leg portion 31, the proximal end side cone region 32, the distal end side cone region 34, and the distal end side leg portion 35).

In the balloon catheter 30 (refer to Fig. 10), it is also acceptable for the proximal ends of the linear members 43 to be joined directly to the outer side tube 21, instead of being joined through the mounting member 21A. In the balloon catheter 40 (refer to Fig. 11), it is also acceptable for the distal ends of the linear members 44 to be joined to the outer circumferential surface 224 of the protruding portion 225 of the inner side tube 22 (refer to Figs. 4 and 6). The proximal ends of the linear members 43 may also be joined to the proximal end side leg portion 31 of the balloon 3. The distal ends of the linear members 43 may also be joined to the distal end side leg portion 35 of the balloon 3. In the balloon catheter 50 (refer to Fig. 12), the bottom portions 51C of the notches 51 (refer to Fig. 13) may also be positioned in the radial direction in substantially the same position as the boundary portion 450B, and may also be positioned radially outward from the boundary portion 450B. In the balloon catheter 50, it is also acceptable for slits to be formed instead of the notches 51. The slits would differ from the notches 51 in that the two faces that are opposite one another in the extension direction would be in contact when the balloon 3 is in the uninflated state. The bottom portions of the slits may also be positioned in the radial direction on one of the inner side of the boundary portion 450B, in substantially the same position as the boundary portion 450B, and on the outer side of the boundary portion 450B. It is also acceptable for the linear members 45 to include neither the notches 51 nor the slits.

## Claims

1. A balloon catheter (10), comprising:
a catheter shaft (2) extending in an extension direction from a proximal end toward a distal end;
a balloon (3) provided on the catheter shaft (2) and inflatable in a radially outward direction centered on the catheter shaft (2);
a linear member (41) disposed on at least an inflation region (33) of an outer circumferential surface (3D) of the balloon (3) and extending in the extension direction, the inflation region (33) extending in the extension direction over a portion of the balloon (3), with a substantially constant diameter throughout the extension direction; and
a pressing member (6) pressing the linear member (41) against the balloon (3), the pressing member (6) including a membrane portion (61, 62) covering at least a portion of the inflation region (33) of the outer circumferential surface (3D) of the balloon (3), the membrane portion (61, 62) including a first membrane portion (61) and a second membrane portion (62), the first membrane portion (61) being in contact with the linear member (41) and directly covering the linear member (41), the second membrane portion (62) being in contact with the outer circumferential surface (3D) of the balloon (3),
wherein
at least a portion of the linear member (41) is disposed between the first membrane portion (61) and the balloon (3) and located outside the second membrane portion (62) in a radial direction with respect to the cross-sectional center of the catheter shaft (2).

2. The balloon catheter (10) according to claim 1, wherein
the membrane portion (61, 62) covers the entirety of the outer circumferential surface (3D) of the balloon (3).

3. The balloon catheter (10) according to claim 1 or 2, wherein
the linear member (41) is provided with
an inflation portion (442) disposed on the inflation region (33) of the balloon (3), and
at least one of a distal end portion positioned toward the distal end from the inflation region (33) and a proximal end portion positioned toward the proximal end from the inflation region (33).

4. The balloon catheter (10) according to any one of claims 1 to 3, wherein
at least a portion of an outer side portion (411) of the linear member (41) is harder than the balloon (3), the outer side portion (411) being disposed on the opposite side of an inner side portion (410), the inner side portion (410) facing the balloon (3).

5. The balloon catheter (10) according to claim 4, wherein
the linear member (41) includes
an extendable soft portion (451) having at least the inner side portion (410), and
a hard portion (452) having at least the outer side portion (411) and having a hardness greater than that of the soft portion (451).

6. The balloon catheter (10) according to any one of claims 1 to 5, wherein
at least a portion of the linear member (41) is joined to the balloon (3).

7. The balloon catheter (10) according to claim 6, wherein
the pressing member (6) includes a joining portion disposed between the balloon (3) and the linear member (41), and
the linear member (41) is joined to the balloon (3) by the joining portion.

8. The balloon catheter (10) according to any one of claims 1 to 7, wherein
at least one of the distal end and the proximal end of the linear member (41) is joined to the catheter shaft (2).

9. A manufacturing method for manufacturing a balloon body (10A), the balloon body (10A) including the balloon (3), the linear member (41), and the pressing member (6) according to any one of claims 1 to 8, and the method comprising processes including:
disposing the linear member (41) on the outer circumferential surface (3D) of the balloon (3);
coating the balloon (3) with a molten substance, with the balloon (3) in a state in which the linear member (41) is disposed on the outer circumferential surface (3D), and the molten substance being the material of the pressing member (6) in a molten state; and
forming the pressing member (6) by drying the molten substance that adheres to the balloon (3) and the linear member (41) after the coating process.

10. A manufacturing method for manufacturing a balloon body (10A), the balloon body (10A) including the balloon (3), the linear member (41), and the pressing member (6) according to any one of claims 1 to 8, and the method comprising processes including:
disposing the pressing member (6) along an inner wall of a die;
disposing the linear member (41) on the opposite side of the pressing member (6) from the die;
injecting a parison, which will become the basis for the balloon (3), on the opposite side of the pressing member (6) from the linear member (41); and
blowing air into the interior of the parison,
wherein
the parison is pressed against the die by the air blown into the interior of the parison, thus forming the balloon (3) and causing the pressing member (6) and the linear member (41) to adhere tightly to the balloon (3).

11. A manufacturing method for manufacturing a balloon body (10A), the balloon body (10A) including the balloon (3), the linear member (41), and the pressing member (6) according to any one of claims 1 to 8, and the method comprising processes including:
disposing the linear member (41) on the outer circumferential surface (3D) of the balloon (3); and
affixing the membranous pressing member (6) to the outer circumferential surface (3D) such that the pressing member (6) covers at least a portion of the linear member (41) from the outer side of the linear member (41).

12. A manufacturing method for manufacturing a balloon body (10A), the balloon body (10A) including the balloon (3), the linear member (41), and the pressing member (6) according to any one of claims 1 to 8, and the method comprising processes including:
disposing the linear member (41) on the outer circumferential surface (3D) of the balloon (3);
covering the linear member (41) from the outer side of the linear member (41) with the pressing member (6), which has a heat shrinking property; and
causing the pressing member (6) to shrink by heating the pressing member (6) in the state in which the linear member (41) is covered by the pressing member (6).

## Patentansprüche

1. Ballonkatheter (10), aufweisend:
einen Katheterschaft (2), der in einer Erstreckungsrichtung vom einem proximalen Ende zu einem distalen Ende verläuft;
einen Ballon (3), der am Katheterschaft (2) vorgesehen und in einer radial nach außen führenden Richtung am Katheterschaft (2) zentriert aufblasbar ist;
ein lineares Element (41), das an mindestens einem Aufblasbereich (33) einer Außenumfangsfläche (3D) des Ballons (3) angeordnet ist und in Erstreckungsrichtung verläuft, wobei sich der Aufblasbereich (33) in Erstreckungsrichtung über einen Teil des Ballons (3) erstreckt, mit einem im Wesentlichen konstanten Durchmesser über die gesamte Erstreckungsrichtung; und
ein Andruckelement (6), welches das lineare Element (41) gegen den Ballon (3) drückt, wobei das Andruckelement (6) einen Membranabschnitt (61, 62) umfasst, der zumindest einen Teil des Aufblasbereichs (33) der Außenumfangsfläche (3D) des Ballons (3) bedeckt, der Membranabschnitt (61, 62) einen ersten Membranabschnitt (61) und einen zweiten Membranabschnitt (62) umfasst, der erste Membranabschnitt (61) in Kontakt mit dem linearen Element (41) steht und das lineare Element (41) direkt bedeckt, und der zweite Membranabschnitt (62) in Kontakt mit der Außenumfangsfläche (3D) des Ballons (3) steht,
wobei
zumindest ein Teil des linearen Elements (41) zwischen dem ersten Membranabschnitt (61) und dem Ballon (3) angeordnet ist und sich außerhalb des zweiten Membranabschnitts (62) in einer radialen Richtung in Bezug auf die Querschnittsmitte des Katheterschafts (2) befindet.

2. Ballonkatheter (10) nach Anspruch 1, wobei
der Membranabschnitt (61, 62) die gesamte Außenumfangsfläche (3D) des Ballons (3) bedeckt.

3. Ballonkatheter (10) nach Anspruch 1 oder 2, wobei
das lineare Element (41) versehen ist mit
einem Aufblasabschnitt (442), der am Aufblasbereich (33) des Ballons (3) angeordnet ist, und
einem distalen Endabschnitt, der in Richtung zum distalen Ende des Aufblasbereichs (33) positioniert ist, und/oder einen proximalen Endabschnitt, der in Richtung zum proximalen Ende des Aufblasbereichs (33) positioniert ist.

4. Ballonkatheter (10) nach einem der Ansprüche 1 bis 3, wobei
mindestens ein Teil eines Außenseitenabschnitts (411) des linearen Elements (41) härter als der Ballon (3) ist, der Außenseitenabschnitt (411) an der entgegengesetzten Seite eines Innenseitenabschnitts (410) angeordnet ist, und der Innenseitenabschnitt (410) dem Ballon (3) zugewandt ist.

5. Ballonkatheter (10) nach Anspruch 4, wobei
das lineare Element (41) umfasst
einen dehnbaren, weichen Abschnitt (451), der mindestens den Innenseitenabschnitt (410) aufweist, und
einen harten Abschnitt (452), der mindestens den Außenseitenabschnitt (411) aufweist und eine Härte hat, die größer als diejenige des weichen Abschnitts (451) ist.

6. Ballonkatheter (10) nach einem der Ansprüche 1 bis 5, wobei
mindestens ein Teil des linearen Elements (41) mit dem Ballon (3) verbunden ist.

7. Ballonkatheter (10) nach Anspruch 6, wobei
das Andruckelement (6) einen Verbindungsabschnitt umfasst, der zwischen dem Ballon (3) und dem linearen Element (41) angeordnet ist, und
das lineare Element (41) über den Verbindungsabschnitt mit dem Ballon (3) verbunden ist.

8. Ballonkatheter (10) nach einem der Ansprüche 1 bis 7, wobei
das distale Ende und/oder das proximale Ende des linearen Elements (41) mit dem Katheterschaft (2) verbunden ist/sind.

9. Herstellverfahren zur Herstellung eines Ballonkörpers (10A), wobei der Ballonkörper (10A) den Ballon (3), das lineare Element (41) und das Andruckelement (6) nach einem der Ansprüche 1 bis 8 aufweist, und das Verfahren Prozesse enthält, die Folgendes umfassen:
Anordnen des linearen Elements (41) an der Außenumfangsfläche (3D) des Ballons (3);
Beschichten des Ballons (3) mit einer geschmolzenen Substanz, wobei der Ballon (3) in einem Zustand ist, in dem das lineare Element (41) an der Außenumfangsfläche (3D) angeordnet ist, und es sich bei der geschmolzenen Substanz um das Material des Andruckelements (6) in einem geschmolzenen Zustand handelt; und
Bilden des Andruckelements (6) unter Trocknung der geschmolzenen Substanz, die am Ballon (3) und am linearen Element (41) anhaftet, und zwar nach dem Beschichtungsprozess.

10. Herstellverfahren zur Herstellung eines Ballonkörpers (10A), wobei der Ballonkörper (10A) den Ballon (3), das lineare Element (41) und das Andruckelement (6) nach einem der Ansprüche 1 bis 8 aufweist, und wobei das Verfahren Prozesse enthält, die Folgendes umfassen:
Anordnen des Andruckelements (6) entlang einer Innenwand eines Formwerkzeugs;
Anordnen des linearen Elements (41) an der dem Formwerkzeug entgegengesetzten Seite des Andruckelements (6);
Einschießen eines Vorformlings, der zur Grundlage für den Ballon (3) wird, an der zum linearen Element (41) entgegengesetzten Seite des Andruckelements (6); und
Einblasen von Luft in das Innere des Vorformlings,
wobei
der Vorformling durch die in das Innere des Vorformlings eingeblasene Luft gegen das Formwerkzeug gedrückt wird, wodurch der Ballon (3) gebildet und bewirkt wird, dass das Andruckelement (6) und das lineare Element (41) eng am Ballon (3) anhaften.

11. Herstellverfahren zur Herstellung eines Ballonkörpers (10A), wobei der Ballonkörper (10A) den Ballon (3), das lineare Element (41) und das Andruckelement (6) nach einem der Ansprüche 1 bis 8 aufweist, und wobei das Verfahren Prozesse enthält, die Folgendes umfassen:
Anordnen des linearen Elements (41) an der Außenumfangsfläche (3D) des Ballons (3); und
Befestigen des membranartigen Andruckelements (6) an der Außenumfangsfläche (3D), derart, dass das Andruckelement (6) von der Außenseite des linearen Elements (41) her mindestens einen Teil des linearen Elements (41) bedeckt.

12. Herstellverfahren zur Herstellung eines Ballonkörpers (10A), wobei der Ballonkörper (10A) den Ballon (3), das lineare Element (41) und das Andruckelement (6) nach einem der Ansprüche 1 bis 8 aufweist, und wobei das Verfahren Prozesse enthält, die Folgendes umfassen:
Anordnen des linearen Elements (41) an der Außenumfangsfläche (3D) des Ballons (3);
Bedecken des linearen Elements (41), von der Außenseite des linearen Elements (41) her, mit dem Andruckelement (6), das eine Wärmeschrumpfeigenschaft hat; und
Bewirken, dass das Andruckelement (6) schrumpft, indem das Andruckelement (6) in dem Zustand erwärmt wird, in dem das lineare Element (41) durch das Andruckelement (6) bedeckt ist.

## Revendications

1. Cathéter à ballonnet (10), comprenant :
un fût de cathéter (2) s'étendant dans une direction d'extension depuis une extrémité proximale vers une extrémité distale ;
un ballonnet (3) disposé sur le fût de cathéter (2) et gonflable dans une direction radialement extérieure centrée sur le fût de cathéter (2) ;
un élément linéaire (41) disposé sur au moins une zone de gonflage (33) d'une surface circonférentielle extérieure (3D) du ballonnet (3) et s'étendant dans la direction d'extension, la zone de gonflage (33) s'étendant dans la direction d'extension sur une partie du ballonnet (3), avec un diamètre sensiblement constant tout au long de la direction d'extension ; et
un élément d'appui (6) appuyant sur l'élément linéaire (41) contre le ballonnet (3), l'élément d'appui (6) incluant une partie membrane (61, 62) recouvrant au moins une partie de la zone de gonflage (33) de la surface circonférentielle extérieure (3D) du ballonnet (3), la partie membrane (61, 62) incluant une première partie membrane (61) et une deuxième partie membrane (62), la première partie membrane (61) étant en contact avec l'élément linéaire (41) et recouvrant directement l'élément linéaire (41), la deuxième partie membrane (62) étant en contact avec la surface circonférentielle extérieure (3D) du ballonnet (3),
sachant que
au moins une partie de l'élément linéaire (41) est disposée entre la première partie membrane (61) et le ballonnet (3) et située à l'extérieur de la deuxième partie membrane (62) dans une direction radiale par rapport au centre en coupe transversale du fût de cathéter (2).

2. Le cathéter à ballonnet (10) selon la revendication 1, sachant que
la partie membrane (61, 62) recouvre l'entièreté de la surface circonférentielle extérieure (3D) du ballonnet (3).

3. Le cathéter à ballonnet (10) selon la revendication 1 ou 2, sachant que
l'élément linéaire (41) est pourvu de
une partie de gonflage (442) disposée sur la zone de gonflage (33) du ballonnet (3), et
au moins l'une d'une partie d'extrémité distale positionnée vers l'extrémité distale de la zone de gonflage (33) et d'une partie d'extrémité proximale positionnée vers l'extrémité proximale de la zone de gonflage (33).

4. Le cathéter à ballonnet (10) selon l'une quelconque des revendications 1 à 3, sachant que
au moins une partie d'une partie latérale extérieure (411) de l'élément linéaire (41) est plus dure que le ballonnet (3), la partie latérale extérieure (411) étant disposée du côté opposé d'une partie latérale intérieure (410), la partie latérale intérieure (410) faisant face au ballonnet (3).

5. Le cathéter à ballonnet (10) selon la revendication 4, sachant que
l'élément linéaire (41) inclut
une partie souple (451) extensible présentant au moins la partie latérale intérieure (410), et
une partie dure (452) présentant au moins la partie latérale extérieure (411) et ayant une dureté supérieure à celle de la partie souple (451).

6. Le cathéter à ballonnet (10) selon l'une quelconque des revendications 1 à 5, sachant que
au moins une partie de l'élément linéaire (41) est assemblée au ballonnet (3).

7. Le cathéter à ballonnet (10) selon la revendication 6, sachant que
l'élément d'appui (6) inclut une partie d'assemblage disposée entre le ballonnet (3) et l'élément linéaire (41), et
l'élément linéaire (41) est assemblé au ballonnet (3) par la partie d'assemblage.

8. Le cathéter à ballonnet (10) selon l'une quelconque des revendications 1 à 7, sachant que
au moins l'une de l'extrémité distale et de l'extrémité proximale de l'élément linéaire (41) est assemblée au fût de cathéter (2).

9. Procédé de fabrication destiné à fabriquer un corps de ballonnet (10A), le corps de ballonnet (10A) incluant le ballonnet (3), l'élément linéaire (41), et l'élément d'appui (6) selon l'une quelconque des revendications 1 à 8, et le procédé comprenant des processus incluant :
la disposition de l'élément linéaire (41) sur la surface circonférentielle extérieure (3D) du ballonnet (3) ;
le revêtement du ballonnet (3) avec une substance fondue, le ballonnet (3) étant dans un état dans lequel l'élément linéaire (41) est disposé sur la surface circonférentielle extérieure (3D), et la substance fondue étant le matériau de l'élément d'appui (6) dans un état fondu ; et
la formation de l'élément d'appui (6) en séchant la substance fondue qui adhère au ballonnet (3) et à l'élément linéaire (41) après le processus de revêtement.

10. Procédé de fabrication destiné à fabriquer un corps de ballonnet (10A), le corps de ballonnet (10A) incluant le ballonnet (3), l'élément linéaire (41), et l'élément d'appui (6) selon l'une quelconque des revendications 1 à 8, et le procédé comprenant des processus incluant :
la disposition de l'élément d'appui (6) le long d'une paroi intérieure d'une matrice ;
la disposition d'un élément linéaire (41) du côté opposé de l'élément d'appui (6) par rapport à la matrice ;
l'injection d'une paraison, laquelle formera la base pour le ballonnet (3), du côté opposé de l'élément d'appui (6) par rapport à l'élément linéaire (41) ; et
le soufflage d'air dans l'intérieur de la paraison,
sachant que
la paraison est appuyée contre la matrice par l'air soufflé dans l'intérieur de la paraison, formant de la sorte le ballonnet (3) et faisant adhérer fermement l'élément d'appui (6) et l'élément linéaire (41) au ballonnet (3).

11. Procédé de fabrication destiné à fabriquer un corps de ballonnet (10A), le corps de ballonnet (10A) incluant le ballonnet (3), l'élément linéaire (41), et l'élément d'appui (6) selon l'une quelconque des revendications 1 à 8, et le procédé comprenant des processus incluant :
la disposition de l'élément linéaire (41) sur la surface circonférentielle extérieure (3D) du ballonnet (3) ; et
la fixation de l'élément d'appui (6) membraneux à la surface circonférentielle extérieure (3D) de telle sorte que l'élément d'appui (6) recouvre au moins une partie de l'élément linéaire (41) depuis le côté extérieur de l'élément linéaire.

12. Procédé de fabrication destiné à fabriquer un corps de ballonnet (10A), le corps de ballonnet (10A) incluant le ballonnet (3), l'élément linéaire (41), et l'élément d'appui (6) selon l'une quelconque des revendications 1 à 8, et le procédé comprenant des processus incluant :
la disposition de l'élément linéaire (41) sur la surface circonférentielle extérieure (3D) du ballonnet (3) ;
le recouvrement de l'élément linéaire (41) depuis le côté extérieur de l'élément linéaire (41) avec l'élément d'appui (6), lequel présente une propriété thermorétrécissable ; et
le fait de provoquer le rétrécissement de l'élément d'appui (6) en chauffant l'élément d'appui (6) dans l'état dans lequel l'élément linéaire (41) est recouvert par l'élément d'appui (6).
